# EUROPEAN PATENT SPECIFICATION

(11) **EP 1 656 381 B1**
(45) Date of publication and mention of the grant of the patent: **09.03.2011**
(21) Application number: 04763994.3
(22) Date of filing: 11.08.2004
(51) Int. Cl.: C07D 495/04

(54) **CRYSTALLISATION OF SOLID FORMS OF CLOPIDOGREL ADDITION SALTS**
KRISTALLISATION VON FESTEN FORMEN VON CLOPIDOGRELADDITIONSSALZEN
CRISTALLISATION DE FORMES SOLIDES DE SELS D'ADDITION DU CLOPIDOGREL

(30) Priority: 13.08.2003 DE 10337773
(43) Date of publication of application: 17.05.2006
(73) Proprietor: Krka, Torvarna Zdravil, D.D., Novo Mesto, 8501 Novo mesto (SI)
(72) Inventor: KOTAR-JORDAN, Berta, 8311 Kostanjevica na Krki (SI); SIMONIC, Igor, 8351 Straza (SI); ZUPET, Rok, 1211 Ljubljana (SI); RUZIC, Milos, 3000 Celje (SI); GRCMAN, Marija, 8212 Velika (SI); PECAVAR, Anica, 8000 Novo mesto (SI)
(74) Representative: UEXKÜLL & STOLBERG
(86) International application number: PCT/EP2004/008989
(87) International publication number: WO 2005/016931

(56) References cited:
- EP-A- 0 281 459
- WO-A-99/65915
- WO-A-03/005162
- WO-A-2004/020443
- WO-A-2004/048385
- WO-A-2004/081016

## Description

### Technical field

The present invention belongs to the field of organic chemistry and relates to a process for preparing crystallized form 1 hydrogen sulfate of clopidogrel. The invention also relates to the use of 2-propylsulphate of clopidogrel and to the use of perchlorate of clopidogrel as intermediate in the preparation of clopidogrel hydrogen sulfate of form 1.

Clopidogrel is the generic name for methyl (+)-(S)-α-(2-chlorophenyl)-6,7-dihydrothieno[3,2-*c*]pyridine-5(4*H*)-acetate and is a compound which has shown to have activity with inhibitory properties towards platelet aggregation, it interferes with the mechanism of formation of arterial and venous thromboses and is useful for the treatment and prevention of platelet disorders.

In the following methyl (+)-(S)-α-(2-chlorophenyl)-6,7-dihydrothieno[3,2-c]pyridine-5(4H)-acetate will be referred to as clopidogrel base whereas clopidogrel is used to refer to the free base as well as addition salts thereof.

### Technical problem

According to prior art processes, the formation as well as the crystallization of the hydrogen sulfate of clopidogrel, in particular of the presently known form 1 hydrogen sulfate of clopidogrel, usually results in a mixture of different polymorphic forms. In particular the preparation of pure clopidogrel hydrogen sulfat of Form 1, i.e. of clopidogrel hydrogen sulfate Form 1 which is substantially free of clopidogrel hydrogen sulfate of Form 2 is difficult to achieve.

In view of the above disadvantages of the prior art processes, there is a need for a crystallization process which leads to a crystalline hydrogen sulfate of clopidogrel having a satisfactory polymorphic purity, and in particular of form 1 hydrogen sulfate of clopidogrel having a high polymorphic purity.

Further, there is a need for precursors which allow the easy preparation of crystalline polymorphic forms of clopidogrel hydrogen sulfate.

These problems are solved by the present invention.

Also disclosed are a solid amorphous form of the hydrogen sulfate of clopidogrel, the 2-propylsulphate of clopidogrel and perchlorate of clopidogrel, as well as processes for their preparation and pharmaceutical compositions comprising them.

### Background of the invention

EP 0 099 802 discloses clopidogrel as well as the platelet anti-aggregating activity of this compound.

EP 0 281 459 describes the existence of two enantiomers of clopidogrel and the crystallized hydrogen sulfate salts thereof with the dextrorotatory isomer having substantially higher therapeutic activity. A process for producing crystallized hydrogen sulfate of clopidogrel involves crystallization from cold acetone.

WO 99/65915 describes two polymorphic forms of clopidogrel hydrogen sulfate, designated form 1 and form 2. Both the form 1 and the form 2 hydrogen sulfate of clopidogrel are characterized by e.g. X-ray data, FT-IR and DSC. The preparation of the form 1 and form 2 clopidogrel hydrogen sulfate is effected by dissolving clopidogrel base in acetone with addition of sulphuric. The mother liquor of the reaction is said to release form 2 clopidogrel hydrogen sulfate after 3 to 6 months. The aqueous-acetone mother liquors contain 0.3 to 1 % of water and about 10% of clopidogrel.

WO 03/051362 discloses crystalline forms III, IV, V and VI of clopidogrel hydrogen sulfate and the amorphous form of clopidogrel hydrogen sulfate, as well as pharmaceutical compositions thereof and processes for preparation of these compounds. Clopidogrel hydrogen sulfate Form III is said to be a solvate of 1-butanol, Form IV of isopropanol, Form V of 2-butanol and Form VI of 1-propanol. The amorphous form of clopidogrel hydrogen sulfate is prepared e.g. by dissolving clopidogrel hydrogen sulfate in acetone and removing the solvent.

### Detailed description of the invention

According to the present invention a process for the preparation of crystallized form 1 hydrogen sulfate of clopidogrel from a solution of hydrogen sulfate of clopidogrel in a solvent is provided, wherein the solvent is
(a) cyclohexanone, ethyl acetate or isopropylacetate, or
(b) a mixture of solvents (a) with at least one other solvent.

The other solvent of the mixture under (b) is selected from the group of solvents in which clopidogrel hydrogen sulfate is soluble, namely methanol, ethanol, acetonitrile, and carboxylic acids such as formic acid and acetic acid.

The preferred solvent mixture is a mixture of isopropyl acetate with methanol.

It was found by the present inventors that the polymorphic form of the product is determined by the choice of solvent, the water content of the solvent and the concentration of the hydrogen sulfate of clopidogrel in the solvent and the temperature.

The solvents and solvent mixtures used in the process according to the invention offer the additional advantage that in the presence of sulfuric acid they do not lead to formation of the undesired by-product mesityl oxide or to formation of water which may have a negative influence on the polymorphic purity of the final product.

The solution of clopidogrel hydrogen sulfate can be obtained in several ways. Firstly, clopidogrel hydrogen sulfate in any form, such as crystalline form 1 or form 2 as well as solid amorphous form, may be dissolved in the selected solvent or solvent mixture. Secondly, clopidogrel base may be reacted with sulphuric acid in the selected solvent or solvent mixture to form a solution of the hydrogen sulfate of clopidogrel. In both cases, the desired final product is obtained by using conventional crystallising such as stirring or partly evaporating solvent and recovering measures such as filtration. If clopidogrel hydrogen sulfate is prepared from the free base by reaction with sulphuric acid, the acid is preferably used in an amount of 1.0 to 1.3, more preferably 1.1 to 1.2 equivalents based on clopidogrel base.

The process according to the invention is suitable to prepare crystallized form 1 hydrogen sulfate of clopidogrel as it allows its preparation with a particularly high polymorphic purity. For this purpose it has been proven preferable that the solvent or solution comprises not more than 1% by weight, preferably not more than 0.5 % by weight and most preferred not more than 0.2 % by weight, of water. Higher amounts of water would favour formation of form 2.

Clopidogrel hydrogen sulfate of form 1 refers to the crystalline form of clopidogrel hydrogen sulfate whose powder X-ray diffractogram shows the following characteristic peaks expressed as interplanar distances at approximately 9.60; 3.49; 3.83; 3.80; 4.31; 8.13; 4.80; 3.86; 5.80 and 4.95 x 10⁻¹⁰ m and whose infrared spectrum exhibits characteristic absorptions expressed in cm⁻¹ at 2987, 1753, 1222, 1175 and 841, as disclosed in EP 1 087 976.

In a preferred embodiment, the process for preparing the form 1 hydrogen sulfate of clopidogrel involves an obtaining of the solution of hydrogen sulfate of clopidogrel by contacting the base of clopidogrel in the selected solvent with sulfuric acid.

In a first embodiment it is preferred that the contacting is effected at a temperature of the solvent or solution of 0 to 50 °C, preferably 22 to 35°C, more preferably 24 to 30 °C. It is further preferred that the dissolving of the base is effected at the same temperature.

In a second embodiment, it is preferred for this purpose that the contacting is effected at a temperature of the solvent or the solution of 0 to 15 °C. It is further preferred that the dissolving of the base is effected at the same temperature.

In both embodiments, the precipitation of crystallized material occurs with effective stirring or seeding.

In a further preferred embodiment, the process for preparing the form 1 hydrogen sulfate of clopidogrel involves an obtaining of the solution of hydrogen sulfate of clopidogrel by dissolving amorphous or crystalline hydrogen sulfate of clopidogrel in the solvent.

It is also preferred that amorphous solid hydrogen sulfate of clopidogrel is dissolved in the solvent. The amorphous solid hydrogen sulfate of clopidogrel has proven particularly useful in the preparation of polymorphic pure form 1 hydrogen sulfate of clopidogrel.

When using the hydrogen sulfate of clopidogrel in the amorphous form, the process according to the invention is preferably carried out by dissolving it in cyclohexanone (at room temperature of solvent) and stirring the solution obtained at room temperature, preferably at 25°C to effect crystallization by the stirring or seeding.

The solution of hydrogen sulfate of clopidogrel may also be obtained by dissolving crystalline hydrogen sulfate of clopidogrel in the solvent. This embodiment of the process is preferably carried out by dissolving the crystalline hydrogen sulfate of clopidogrel in methanol, formic or acetic acid, partly evaporating the solvent, diluting the residual mixture with ketone or ester as defined above and stirring the diluted mixture at e.g. 0 to 15°C to effect formation of crystalline form 1 material.

For preparing clopidogrel hydrogen sulfate of form 1 the concentration of clopidogrel hydrogen sulfate is preferably within a range of 20 to 60 % by weight, more preferably 25 to 50 % by weight.

Generally, the form 1 hydrogen sulfate of clopidogrel is rather difficult to be prepared in substantially pure form, because the formation of the thermodynamically more stable form 2 is favoured. However, by using the above process according to the invention it was possible to prepare form 1 hydrogen sulfate of clopidogrel not only in high yields of above 90%, but also with a high polymorphic purity. Thus, the process according to the present invention allows the producing of substantially pure form 1 hydrogen sulfate of clopidogrel free from form 2 and solvates.

surprisingly found out that for formation of the form 2 hydrogen sulfate it is preferred that the solvent or solution comprises more than 1% by weight of water and more than 10 % by weight of hydrogen sulfate of clopidogrel. It is preferred to use these conditions and to effect the crystallization at room temperature; no use of seeding crystals of form 2 hydrogen sulfate of clopidogrel is necessary. The product usually crystallises within an one hour or less with effective stirring. The water content is preferably below 10 % by weight.

Clopidogrel hydrogen sulfate of form 2 refers to the crystalline form of clopidogrel hydrogen sulfate whose powder X-ray diffractogram shows the following characteristic peaks expressed as interplanar distances at approximately 4.11; 6.86; 3.60; 5.01; 3.74; 6.49 and 5.66 x 10⁻¹⁰ m and whose infrared spectrum exhibits characteristic absorptions expressed in cm⁻¹ at 2551, 1497, 1189, and 1029, as disclosed in EP 1 087 976.

The crystallisation of form 2 is in particular effected in 2-propanol and the mixture thereof with methanol, ethyl acetate or acetone. In case of use of a mixture with methanol or other solvents the crystallization is preferably carried out at temperatures of the solution of 10 to 60°C, preferably 20 to 60 °C, the most preferably 30 to 60 °C.

Also disclosed is a process for preparing the amorphous solid form of the hydrogen sulfate of clopidogrel. The amorphous solid hydrogen sulfate of clopidogrel provides benefits when producing in particular morphologically pure form 1 hydrogen sulfate of clopidogrel.

In the process for preparing the amorphous solid hydrogen sulfate
(a) the hydrogen sulfate of clopidogrel is dissolved in a suitable solvent,
(b) the solution obtained is diluted with water, and
(c) the diluted solution is spray dried or lyophilized.

Preferred solvents are water miscible solvents, in particular C₁-C₄ alkyl alcohols, preferably methanol and ethanol, and C₁-C₃ carboxylic acids, preferably formic acid or acetic acid.

Another preferred process for preparing the amorphous solid hydrogen sulfate involves the steps of
(a) dissolving the hydrogen sulfate of clopidogrel in a solvent or a mixture of solvents, preferably, methanol, 2-propanol, isobutyl methyl ketone or heptane or a mixture thereof,
(b) optionally removing a part of the solvent or solvent mixture,
(c) preferably adding another solvent or solvent mixture, preferably a solvents selected from the group consisting of 2-propanol, acetone, isobutyl methyl ketone, heptane and mixtures thereof, and
(d) evaporating all solvents, preferably under reduced pressure, or filtering the mixture of step (b) or (c) to isolate the amorphous product.

It is preferred to use methanol in step (a). The solution of step (a) may be purified by filtration before proceeding further with the process. It is further preferred to perform at least steps (a), (c) and (d).

The amorphous solid form of clopidogrel hydrogen sulfate is stable at temperatures under 10°C, while higher temperatures or grinding at temperatures of 5°C or more causes gradually a transformation to form 1 and to form 2 hydrogen sulfate of clopidogrel.

The amorphous state of the substance was confirmed by X-ray data.

The amorphous form of clopidogrel hydrogen sulfate is particularly useful for the production of form 1 clopidogrel hydrogen sulfate. Thus, a preferred process of the present invention for preparing clopidogrel hydrogen sulfate form 1 involves the steps of
(1) preparing amorphous solid clopidogrel hydrogen sulphate, and
(2) converting the amorphous solid clopidogrel hydrogen sulphate of step (1) to form 1 clopidogrel hydrogen sulphate.

Both step (1) and step (2) are preferably performed as described above.

The processes of the present invention give clopidogrel hydrogen sulfate of form 1 which is substantially free of form 2, i.e. contains less than 5 % by weight, preferably less than 3 % by weight, more preferably less than 1 % by weight of form 2. In addition the product preferably contains less than 1.0 % by weight, more preferably less than 0.5 % by weight and most preferably less than 0.2 % by weight of the R isomer of clopidogrel.

Finally, novel salts of clopidogrel, namely the 2-propylsulphate of clopidogrel **(1)** and perchlorate of clopidogrel **(2)** in accordance with the following formula, are disclosed

2-propylsulphate of clopidogrel **(1)** 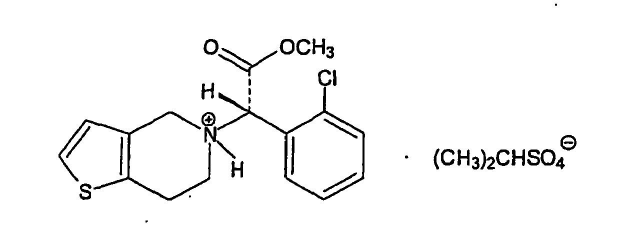 perchlorate of clopidogrel **(2)**

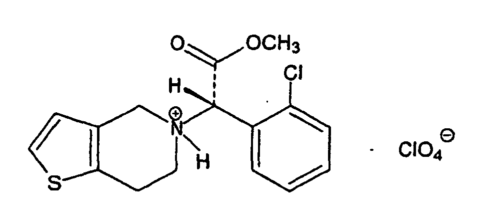

The structure of 2-propylsulphate of clopidogrel **(1)** is confirmed by elemental analysis : calc. 49,29%C, 5,44%H, 3,02%N, found 48,23%C, 5,55%H, 2,89%N, ¹H-NMR spectroscopic data (300MHz, solvent DMSO-d₆, in ppm) :1,11 (d, 6H,2Me i-propyl, 3,1 - 4,3 (m, tetrahydropyridinic ring), 3,76 (s, 3H, COOMe),4,3(q, 1H, CH i-propyl), 5.64 (s, 1H, N-CH=), 6.89 (d, 1H, thiophenic ring), 7.45 (d, 1H, thiophenic ring), 7.50 - 7.60 (m, 2H, Ph), 7.65 - 7.72 (m, 2H, Ph), MS: FAB+ 322 MH of clopidogrel and FAB- 139 of 2-propylsulphate. The compound **1** has a melting point of 170 ± 2°C.

The compound **2** has a melting point of 165 - 170°C and is further characterized by the following ¹H-NMR spectroscopic data (300MHz, solvent DMSO-d₆, in ppm): 3.2 - 4.5 (m, tetrahydropyridinic ring), 3.76 (s, 3H, COOMe), 5.604 (s, 1H, N-CH=), 6.89 (d, 1H, thiophenic ring), 7.44 (d, 1H, thiophenic ring), 7.50 - 7.60 (m, 2H, Ph), 7.64 - 7.72 (m, 2H, Ph).

A preferred process for preparing the 2-propylsulphate of clopidogrel involves the steps of
(a) dissolving or suspending hydrogen sulfate of clopidogrel in 2-propanol,
(b) heating the obtained solution or suspension until the boiling point for about 2 hours to conversion all hydrogen sulphate to 2-propyl sulphate,
(c) crystallisation occurs with cooling or partly evaporating of solvent.

Step (a) is preferably performed at room temperature. In step (b) the solution or suspension is heated under reflux until HPLC analysis indicates that the educt has totally reacted. Heating is preferably continued for a time period of 1 to 3 hours.

Another preferred process for preparing the 2-propylsulphate of clopidogrel involves the steps of
(a) dissolving clopidogrel (base) in 2-propanol with sulphuric acid at room temperature,
(b) heating the obtained solution or suspension until the boiling point,
(c) crystallisation occurs with cooling or partly evaporating of solvent or with addition of anti-solvent such as heptane.

In step (a) the free base is converted to the hydrogen sulfate addition salt of clopidogrel. In step (b) the mixture is heated until all of hydrogen sulfate has been converted to the corresponding 2-propylsulphate as indicated above.

A preferred process for preparing the perchlorate of clopidogrel comprises the following steps:
(a) methyl (+)-(S)-alpha- (o-chlorophenyl)-6,7-dihydrothieno[3,2-c]pyridine-5(4H)-acetate is dissolved in a solvent or a mixture of solvents, preferably ethyl acetate, 2-propanol, ethanol or a mixture thereof,
(b) to the obtained solution perchloric acid is added in equimolar amount or in excess,
(c) the product crystallises from the obtained solution.

Compounds (1) and (2) can be used as intermediates in the preparation of crystallised forms of clopidogrel sulphate in particular clopidogrel sulphate of form 1.

Both the 2-propylsulphate salt and the perchlorate salt of clopidogrel hydrogen sulfate can be formulated in a pharmaceutical composition. The invention therefore also provides a pharmaceutical composition comprising the 2-propylsulphate of clopidogrel, the perchlorate of clopidogrel or the amorphous solid clopidogrel hydrogen sulfate and at least one pharmaceutically acceptable carrier or excipient.

The invention is in the following further illustrated by examples.

### Examples

### Example 1: Preparation of clopidogrel hydrogen sulfate form 1 (illustrative)

0.80 kg of 2-propylsulfate of clopidogrel were prepared as described in Example 9 and then suspended in 6.4 1 of ethyl acetate and 2.4 1 of water at room temperature. 2 L of 1M NaOH was added and adjusted pH to 7.5-8.0. After stirring, the organic phase was separated off and washed twice with water. The solvent was evaporated off under vacuum at 50°C and the residue dissolved in 2.2 1 of 2-propanol, the solvent evaporated under vacuum at 25°C to the oily residue (0.45 kg). To the resulting oily residue was added the solution of 74.4 ml 98% sulphuric acid in 1.5 1 of 2-propanol at 25-30°C under nitrogen. The resulting solution was seeded with crystals of form I and stirred at 25°C. The water content of the reaction mixture was 0.3 % as determined by the Karl Fischer method. The crystals obtained were filtered off and dried in a vacuum at 50°C (0.49 kg).

### Example 2: Preparation of clopidogrel hydrogen sulfate form 1 (illustrative)

42.4 g of clopidogrel hydrogen sulfate form 2 was suspended in 340 ml of methylene chloride, then 226 ml of a saturated aqueous solution of sodium hydrogen carbonate was added to obtain alkaline solution. The organic phase was separated, washed with water and dried with magnesium sulphate. The methylene chloride solution was concentrated, 120 ml 2-propanol was added and the solvent evaporated to the resulting oily residue. The oil was dissolved in 128 ml of 2-propanol at room temperature and 5.5 ml of concentrated (98%) sulphuric acid was added drop-wise at 24 to 28°C under nitrogen. The water content of the mixture was 0.2 % as determined by the Karl Fischer method. The resulting solution was seeded with crystals of form I and stirred at 25°C. The crystals obtained were filtered off and dried in a vacuum at 50°C (38.0 g).

This example was repeated using clopidogrel hydrogen sulfate of form 1, a mixture of clopidogrel hydrogen sulfate form 1 and form 2 or amorphous clopidogrel hydrogen sulfate instead of clopidogrel hydrogen sulfate form 2. The products were all identical to the product obtained with clopidogrel hydrogen sulfate form 2.

### Example 3: Preparation of clopidogrel hydrogen sulfate form 1 by crystallisation from 2-propanol (illustrative)

100.0 g of clopidogrel hydrogen sulfate of form 2 was dissolved in 150 ml of methanol, the solution was filtered and 240 ml of acetone was added. The obtained solution was evaporated under vacuum at 30 - 35°C to dryness (125.2 g of amorphous clopidogrel hydrogen sulfate).

10.0 g of the amorphous solid clopidogrel hydrogen sulfate were then dissolved in 30 ml of 2-propanol and mixed at room temperature for three hours. The water content of the mixture was 0.2 % as determined by the Karl Fischer method. The product was filtered and dried under vacuum at 50°C (7.7 g).

### Example 4: Preparation of clopidogrel hydrogen sulfate form 1 by crystallisation from methanol/2-propanol (illustrative)

10.0 g of clopidogrel hydrogen sulfate of form 2 was dissolved in 15 ml of methanol, the solution was filtered and concentrated to about 12.5 g. The concentrated oily residue contained 20 % by weight of methanol. The residue was dissolved in 30 ml of cold 2-propanol solvent and stirred at 10-12°C. The water content of the reaction mixture was 0.2 to 0.3 % as determined by the Karl Fischer method. The product was isolated by filtration and dried in a vacuum at 50°C (8.7 g).

This example was repeated using clopidogrel hydrogen sulfate of form 1, a mixture of clopidogrel hydrogen sulfate form 1 and form 2 or amorphous clopidogrel hydrogen sulfate instead of clopidogrel hydrogen sulfate form 2. The products were all identical to the product obtained with clopidogrel hydrogen sulfate form 2.

### Example 5: Preparation of clopidogrel hydrogen sulfate form 1 by crystallisation from methanol/2-propanol (illustrative)

1.0 g of clopidogrel hydrogen sulfate of form 2 or was dissolved in 1.2 ml of methanol, then 10 ml of 2-propanol with 0.1 g of form 1 hydrogen sulfate of clopidogrel was added to the methanol solution. The water content of the reaction mixture was 0.2 % as determined by the Karl Fischer method. The mixture was cooled and left to stand at -5 to 0°C. The precipitate formed was filtered and dried in a vacuum at 50°C (0.4 g).

This example was repeated using clopidogrel hydrogen sulfate of form 1, a mixture of clopidogrel hydrogen sulfate form 1 and form 2 or amorphous clopidogrel hydrogen sulfate instead of clopidogrel hydrogen sulfate form 2. The products were all identical to the product obtained with clopidogrel hydrogen sulfate form 2.

### Example 6: Preparation of clopidogrel hydrogen sulfate form 2 (illustrative)

10.0 g of clopidogrel hydrogen sulfate of form 1 was dissolved in 15 ml of methanol, the solution was filtered and concentrated to about 12.5 g. The residue contained 20 % by weight of methanol. The residue was dissolved in 30 ml of cold 2-propanol and stirred at 25°C. The product was isolated by filtration and dried in a vacuum at 50°C (8.9 g).

This example was repeated using clopidogrel hydrogen sulfate of form 2, a mixture of clopidogrel hydrogen sulfate form 1 and form 2 or amorphous clopidogrel hydrogen sulfate instead of clopidogrel hydrogen sulfate form 1. The products were all identical to the product obtained with clopidogrel hydrogen sulfate form 1.

### Example 7: Preparation of amorphous clopidogrel hydrogen sulfate

15.0 g of clopidogrel hydrogen sulfate of form 2 or was dissolved in 150 ml of methanol and 200 ml of water was added. The obtained solution was spray dried by using a spray dryer, namely mini spray-dryer Büchi 190. The product was dried under vacuum at 25°C (3.0 g).

The reaction was repeated using clopidogrel hydrogen sulfate of form 1 or a mixture of form 1 and form 2 instead of clopidogrel hydrogen sulfate form 2. In both instances amorphous Clopidogrel hydrogen sulfate was obtained.

### Example 8: Preparation of amorphous clopidogrel hydrogen sulfate

10.0 g of clopidogrel hydrogen sulfate of form 2 was dissolved in 15 ml of methanol and 100 ml of water was added. The obtained solution was lyophilized. The product was dried under vacuum at 25°C (9.7 g).

### Example 9: Preparation of the 2-propylsulphate of clopidogrel

20 g of clopidogrel hydrogen sulfate was suspended in 200 ml of 2-propanol, heated to reflux in 30 minutes and another 200 ml of 2-propanol was added. The suspension was stirred for about 1 hour to obtain a clear solution. The reaction mixture was divided into three different portions which were further processed at different temperatures:
a)110 ml of that solution was cooled 5°C and stirred at that temperature for 2 hours. The product was recovered by filtration and dried under vacuum at 30 °C (3.55 g).
b)110 ml of that solution was gradually cooled to room temperature and stirred at that temperature for 3 hours and cooled to 5°C. The product was recovered by filtration and dried under vacuum at 30 °C (4.8 g).
c)220 ml of that solution was partly concentrated under vacuum at 50°C and stirred at room temperature for 2 hours. The product was recovered by filtration and dried under vacuum at 30 °C (9.54 g).

### Example 10: Preparation of the 2-propylsulphate of clopidogrel

16.4 g of clopidogrel (base) was dissolved in 75 ml of 2-propanol and 2.75 ml of 98% sulphuric acid was added at 25 to 28°C. The solution was gradually heated to 50°C, diluted with 60 ml of 2-propanol and heated to the boiling temperature with stirring for 2 hours. The clear solution was cooled in 1 hour to room temperature and obtained crystals stirred for 1 hour. The product was recovered by filtration to yield 16.1 g.

### Example 11: Preparation of the perchlorate of clopidogrel

13.85 g of methyl (+)-alpha-(2-thienylethylamino) (2-chlorophenyl)acetate hydrochloride was suspended in 40 ml of 37% aqueous solution of formaldehyde. After 3.5 hours stirring at 45°C, the reaction mixture was cooled to room temperature. 10 ml of water, 150 ml of ethyl acetate and 60 ml of 5 % aqueous sodium hydrogen carbonate solution were introduced into reaction mixture. After stirring, the organic phase was separated off and washed with 5 % aqueous sodium hydrogen sulfite and water. The solvent was evaporated off under vacuum and the residue dissolved in 60 ml of 2-propanol. To the mixture 3,5 ml of 70% aqueous perchloric acid was slowly added at room temperature (optionally under nitrogen) and mixed for 1 hour. The crystals were filtered off. After drying in vacuum at 50°C 14.2 g of the product was obtained.

### Example 12: Preparation of clopidogrel hydrogen sulfate form 1 (illustrative)

The solution of sulphuric acid (16.4 ml, 0.306 mole) in 108 ml 2-propanol was added to 86.6 g of clopidogrel base (0.25 mole, assay by HPLC) following with effective agitation. The warm solution was cooled to 30°C and seeded with activating seeding with 2 g of form 1 in 2-propanol. The crystallisation was carried out at 29-27°C for two hours, then gradually cooled to 22°C.

The product was filtered and partly dried in an vacuum oven at room temperature. 104 g of that product was suspended in 300 ml of 2-propanol at 8°C, mixed 10 minutes, filtered and dried in vacuum drier at 50°C to the constant weight. Yield: 90.3 g.

### Example 13: Preparation of clopidogrel hydrogen sulfate form 1 (illustrative)

100 g of clopidogrel hydrogen sulfate was dissolved in 300 ml of methanol at room temperature, filtered to obtained clear solution and concentrated at vacuum approximately to 1/3 of total volume. The evaporation was continued with 200 ml of 2-propanol to obtain 156 g of oily product. Finally, that product was dissolved in 150 ml of 2-propanol with effective agitation at 35°C, cooled to 30°C, seeded with 1 g of form 1 in 2-propanol. The water content of the mixture was 0.2 % as determined by the Karl Fisher method. The crystallisation was carried out at 29-27°C for two hours, then gradually cooled to 22°C.

The product was filtered and partly dried in an vacuum oven at room temperature. 94 g of that product was suspended in 180 ml of 2-propanol at 5°C, mixed 10 minutes, filtered and dried in vacuum drier at 50°C to the constant weight. Yield: 95.6 g

### Example 14: Preparation of clopidogrel hydrogen sulfate form 1

1.0 g of clopidogrel hydrogen sulfate (amorphous form) was dissolved in 3 ml of cyclohexanone and mixed at room temperature three hours. The product was filtered and dried under vacuum at 50°C (0.4 g).

### Example 15: Preparation of clopidogrel hydrogen sulfate form 1 (illustrative)

1.0 g of clopidogrel hydrogen sulfate (amorphous form) was suspended in 3 ml of heptane and mixed at room temperature one day. The product was filtered and dried in a vacuum at 50 °C.

### Example 16: Preparation of amorphous clopidogrel hydrogen sulfate

10.0 g of clopidogrel hydrogen sulfate of form 1 was dissolved in 12 ml of methanol and 20 ml of 2-propanol was added. The obtained solution was evaporated under vacuum at 30 - 35°C to dryness (10.0 g).

### Example 17: Preparation of amorphous clopidogrel hydrogen sulfate

100.0 g of clopidogrel hydrogen sulfate of form 2 was dissolved in 150 ml of methanol, the solution was filtered and 240 ml of acetone was added. The obtained solution was evaporated under vacuum at 30 - 35°C to dryness (125.2 g).

### Example 18: Preparation of amorphous clopidogrel hydrogen sulfate

2.0 g of clopidogrel hydrogen sulfate of form 1 was dissolved in 5 ml of methanol, the solvent was partly evaporated under vacuum at 35°C. A mixture of acetone, isobutylmethyl ketone and heptane (3 ml) was added to the resulting oil and it was stirred at room temperature. The product was separated by filtration and dried under vacuum at 30 °C (1.75 g).

### Example 19: Preparation of amorphous clopidogrel hydrogen sulfate

2.0 g of clopidogrel hydrogen sulfate of form 1 was dissolved in 5 ml of methanol, the solvent was partly evaporated under vacuum at 35°C. A mixture of acetone, isobutylmethyl ketone and heptane (3 ml, 1:1:1) was added to the resulting oil and it was stirred at room temperature. The product was recovered by filtration and dried under vacuum at 30°C (1.75 g).

### Example 20: Preparation of clopidogrel hydrogen sulfate form 1

1.0 g of clopidogrel hydrogen sulfate of form 2 was dissolved in 1 ml of methanol and added to a mixture of clopidogrel hydrogen sulfate form 1 (50 mg) in isopropyl acetate (15 ml). The resulting mixture was stirred overnight at room temperature. The solid formed was crushed and stirred for additional 30 minutes. Polymorph I was isolated by filtration and drying in a vacuum at 30°C (0.87 g).

This procedure was repeated using clopidogrel hydrogen sulfate of form 1, a mixture of clopidogrel hydrogen sulfate form 1 and form 2 or amorphous clopidogrel hydrogen sulfate instead of clopidogrel hydrogen sulfate form 2. The products were all identical to the product obtained with clopidogrel hydrogen sulfate form 2.

The procedure was also repeated using n-butyl acetate instead of isopropyl acetate. The product was identical to the product obtained with isopropyl acetate.

## Claims

1. Process for preparing crystallized form 1 hydrogen sulfate of clopidogrel from a solution of hydrogen sulfate of clopidogrel in a solvent, wherein the solvent is
(a) cyclohexanone, ethyl acetate or isopropyl acetate, or
(b) a mixture of solvents (a) with at least one other solvent, wherein the other solvent of mixture (b) is selected from the group of methanol, ethanol, acetonitrile and carboxylic acids,
wherein form 1 hydrogen sulfate of clopidogrel is a crystalline form of clopidogrel hydrogen sulfate whose powder X-ray diffractogram shows characteristic peaks expressed as interplanar distances at approximately 9.60; 3.49; 3.83; 3.80; 4.31; 8.13; 4.80; 3.86; 5.80 and 4.95 x 10⁻¹⁰ m and whose infrared spectrum exhibits characteristic absorptions expressed in cm⁻¹ at 2987, 1753, 1222, 1175 and 841.

2. Process according to claim 1, wherein the solution of hydrogen sulfate of clopidogrel is obtained by contacting methyl (+)-(S)-alpha-(o-chlorophenyl)-6,7-dihydrothieno[3,2-c]pyridine-5(4H)-acetate in the solvent with sulfuric acid.

3. Process for preparing clopidogrel hydrogen sulfate according to any one of claims 1 or 2, wherein solvent (a) or solvent mixture (b) comprises not more than 1 % by weight of water.

4. Process according to claim 2 or 3, wherein the contacting is effected at a temperature of the solvent of 0 to 50°C, preferably 22 to 35 °C, more preferably 24 to 30 °C.

5. Process according to claim 3, wherein the solution of hydrogen sulfate of clopidogrel is obtained by dissolving hydrogen sulfate of clopidogrel in the solvent.

6. Process according to claim 5, wherein amorphous solid hydrogen sulfate of clopidogrel is dissolved in the solvent.

7. Process according to any one of claims 3 to 6, wherein the solvent or solution comprises not more than 0.5 % by weight and preferred not more than 0.2 % by weight, of water.

8. Process according to claim 3, comprising the steps
(1) preparing amorphous solid clopidogrel hydrogen sulfate, and
(2) converting the amorphous solid clopidogrel hydrogen sulfate to form 1 clopidogrel hydrogen sulfate.

9. Process according to claim 8, wherein in step (1) the amorphous solid clopidogrel hydrogen sulfate is prepared according to a process, wherein
(a) the hydrogen sulfate of clopidogrel is dissolved in a water miscible organic solvent
(b) the solution obtained is diluted with water, and
(c) the diluted solution is spray-dried or lyophilized.

10. Process according to claim 8 or 9, wherein in step (2) the amorphous solid clopidogrel hydrogen sulfate is converted to form 1 clopidogrel hydrogen sulfate by the process of any one of claims 5 to 7.

11. Use of the 2-propylsulphate of clopidogrel of the following formula 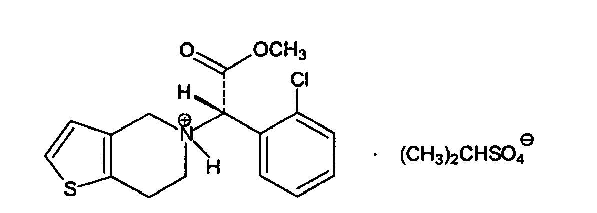 or the perchlorate of clopidogrel of the following formula 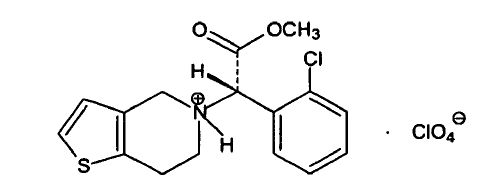 as intermediate in the preparation of clopidogrel hydrogen sulfate of form 1.

12. Use according to claim 11, wherein the crystallization is effected by the process according to any one of claims 1 to 7.

13. Process for the preparation of clopidogrel hydrogen sulfate of form 1, wherein the 2-propylsulphate of clopidogrel of the following formula 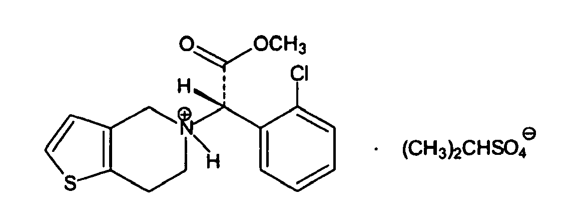 or the perchlorate of clopidogrel of the following formula 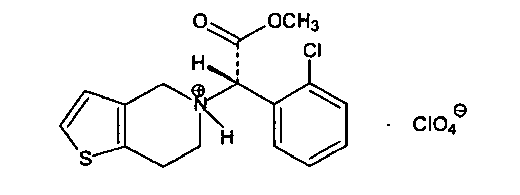 is used as intermediate.

14. Process according to claim 13, wherein the crystallization is effected by the process according to any one of claims 1 to 7.

## Patentansprüche

1. Verfahren zur Herstellung von kristallisierter Form 1 des Hydrogensulfats von Clopidogrel aus einer Lösung des Hydrogensulfats von Clopidogrel in einem Lösungsmittel, wobei das Lösungsmittel
(a) Cyclohexanon, Ethylacetat oder Isopropylacetat, oder
(b) eine Mischung der Lösungsmittel (a) mit mindestens einem weiteren Lösungsmittel ist, wobei das weitere Lösungsmittel der Mischung (b) aus der Gruppe aus Methanol, Ethanol, Acetonitril und Carbonsäuren ausgewählt ist,
wobei die Form 1 des Hydrogensulfats von Clopidogrel eine kristalline Form von Clopidogrelhydrogensulfat ist, dessen Pulver-Röntgendiffraktogramm charakteristische Peaks, ausgedrückt als interplanare Entfernungen, bei etwa 9,60, 3,49, 3,83, 3,80, 4,31, 8,13, 4,80, 3,86, 5,80 und 4,95 x 10⁻¹⁰ m zeigt, und dessen Infrarotspektrum charakteristische Absorptionen, ausgedrückt in cm⁻¹, bei 2987, 1753, 1222, 1175 und 841 aufweist.

2. Verfahren nach Anspruch 1, bei dem die Lösung des Hydrogensulfats von Clopidogrel erhalten wird, indem Methyl-(+)-(S)-alpha-(o-chlorphenyl)-6,7-dihydrothien[3,2-c]pyridin-5(4H)-acetat in dem Lösungsmittel mit Schwefelsäure in Kontakt gebracht wird.

3. Verfahren zur Herstellung von Clopidogrelhydrogensulfat nach einem der Ansprüche 1 oder 2, bei dem das Lösungsmittel (a) oder die Lösungsmittelmischung (b) nicht mehr als ein 1 Gew.-% Wasser enthält.

4. Verfahren nach Anspruch 2 oder 3, bei dem das in Kontakt bringen bei einer Temperatur des Lösungsmittels von 0 bis 50 °C, vorzugsweise 22 bis 35 °C, bevorzugter 24 bis 30°C durchgeführt wird.

5. Verfahren nach Anspruch 3, bei dem die Lösung des Hydrogensulfats von Clopidogrel erhalten wird, indem Hydrogen-sulfat von Clopidogrel in dem Lösungsmittel gelöst wird.

6. Verfahren nach Anspruch 5, bei dem amorphes, festes Hydrogensulfat von Clopidogrel in dem Lösungsmittel gelöst wird.

7. Verfahren nach einem der Ansprüche 3 bis 6, bei dem das Lösungsmittel oder die Lösung nicht mehr als 0,5 Gew.-% und vorzugsweise nicht mehr als 0,2 Gew.-% Wasser enthält.

8. Verfahren nach Anspruch 3, bei dem
(1) amorphes, festes Clopidogrelhydrogensulfat hergestellt wird, und
(2) das amorphe, feste Clopidogrelhydrogensulfat unter Bildung von Clopidogrelhydrogensulfat der Form 1 umgesetzt wird.

9. Verfahren nach Anspruch 8, bei dem in Stufe (1) das amorphe, feste Clopidogrelhydrogensulfat gemäß einem Verfahren hergestellt wird, bei dem
(a) das Hydrogensulfat von Clopidogrel in wassermischbarem organischen Lösungsmittel gelöst wird,
(b) die erhaltene Lösung mit Wasser verdünnt wird, und
(c) die verdünnte Lösung sprühgetrocknet oder lyophilisiert wird.

10. Verfahren nach Anspruch 8 oder 9, bei dem in Stufe (2) das amorphe feste Clopidogrelhydrogensulfat durch das Verfahren gemäß einem der Ansprüche 5 bis 7 in Clopidogrelhydrogensulfat der Form 1 überführt wird.

11. Verwendung des 2-Propylsulfats von Clopidogrel der folgenden Formel 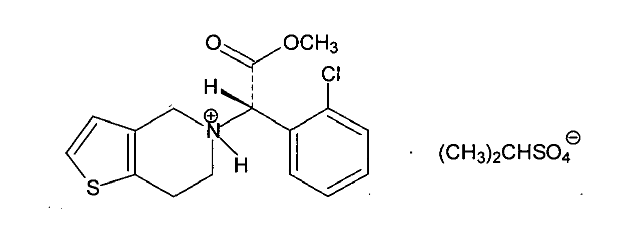 oder des Perchlorats von Clopidogrel der folgenden Formel 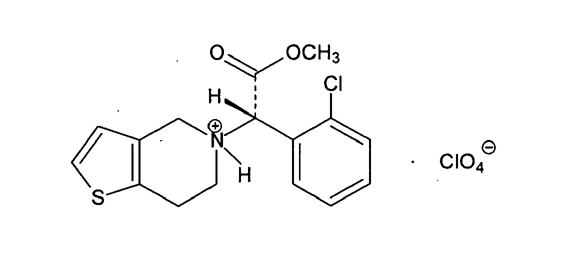 als Zwischenprodukt bei der Herstellung von Clopidogrelhydrogensulfat der Form 1.

12. Verwendung nach Anspruch 11, bei dem die Kristallisation durch das Verfahren gemäß einem der Ansprüche 1 bis 7 durchgeführt wird.

13. Verfahren zur Herstellung von Clopidogrelhydrogensulfat der Form 1, bei dem das 2-Propylsulfat von Clopidogrel der folgenden Formel 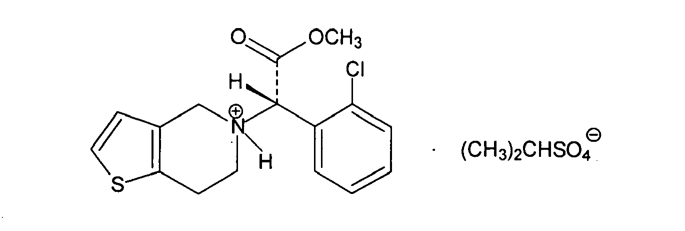 oder das Perchlorat von Clopidogrel der folgenden Formel 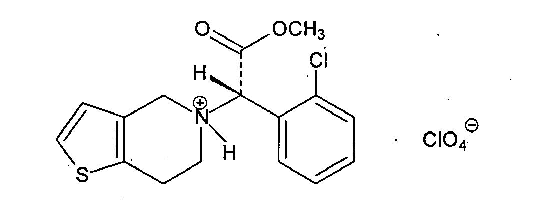 als Zwischenprodukt verwendet wird.

14. Verfahren nach Anspruch 13, bei dem die Kristallisation durch das Verfahren gemäß einem der Ansprüche 1 bis 7 durchgeführt wird.

## Revendications

1. Procédé de préparation de la forme cristallisée 1 de l'hydrogénosulfate de clopidogrel à partir d'une solution d'hydrogénosulfate de clopidogrel dans un solvant, dans lequel procédé le solvant est
a) de la cyclohexanone, de l'acétate d'éthyle ou de l'acétate d'iso-propyle,
b) ou un mélange d'un solvant (a) et d'au moins un autre solvant, lequel autre solvant de mélange (b) est choisi dans l'ensemble formé par le méthanol, l'éthanol, l'acétonitrile et les acides carboxyliques,
la forme 1 de l'hydrogénosulfate de clopidogrel étant une forme cristalline d'hydrogénosulfate de clopidogrel dont le diffractogramme de poudre aux rayons X présente des pics caractéristiques correspondant à des distances interplanaires d'environ 9,60, 3,49, 3,83, 3,80, 4,31, 8,13, 4,80, 3,86, 5,80 et 4,95 .10⁻¹⁰ m et dont le spectre d'absorption infrarouge présente des absorptions caractéristiques à 2987, 1753, 1222, 1175 et 841 cm⁻¹.

2. Procédé conforme à la revendication 1, dans lequel on obtient la solution d'hydrogénosulfate de clopidogrel en mettant du (+)-(S)-alpha-(ortho-chloro-phényl)-6,7-dihydro-thiéno[3,2-c]pyridine-5(4H)-acétate de méthyle en contact avec de l'acide sulfurique, dans le solvant.

3. Procédé de préparation d'hydrogénosulfate de clopidogrel, conforme à la revendication 1 ou 2, dans lequel le solvant (a) ou le mélange (b) de solvants ne contient pas plus de 1 % en poids d'eau.

4. Procédé conforme à la revendication 2 ou 3, dans lequel on réalise la mise en contact alors que le solvant est à une température de 0 à 50 °C, de préférence de 22 à 35 °C, et mieux encore de 24 à 30 °C.

5. Procédé conforme à la revendication 3, dans lequel on obtient la solution d'hydrogénosulfate de clopidogrel en dissolvant de l'hydrogénosulfate de clopidogrel dans le solvant.

6. Procédé conforme à la revendication 5, dans lequel c'est de l'hydrogénosulfate de clopidogrel à l'état de solide amorphe qu'on dissout dans le solvant.

7. Procédé conforme à l'une des revendications 3 à 6, dans lequel le solvant ou la solution ne contient pas plus de 0,5 % en poids d'eau, et de préférence, ne contient pas plus de 0,2 % en poids d'eau.

8. Procédé conforme à la revendication 3, comportant les étapes suivantes :
1) préparer de l'hydrogénosulfate de clopidogrel à l'état de solide amorphe ;
2) et convertir cet hydrogénosulfate de clopidogrel solide amorphe en de la forme 1 d'hydrogénosulfate de clopidogrel.

9. Procédé conforme à la revendication 8, dans l'étape (1) duquel on prépare de l'hydrogénosulfate de clopidogrel à l'état de solide amorphe en opérant selon un procédé dans lequel
a) on dissout de l'hydrogénosulfate de clopidogrel dans un solvant organique miscible à l'eau,
b) on dilue avec de l'eau la solution ainsi obtenue,
c) et l'on opère sur cette solution diluée un séchage par pulvérisation ou une lyophilisation.

10. Procédé conforme à la revendication 8 ou 9, dans l'étape (2) duquel c'est par un procédé conforme à l'une des revendications 5 à 7 que l'on convertit l'hydrogénosulfate de clopidogrel solide amorphe en de la forme 1 d'hydrogénosulfate de clopidogrel.

11. Utilisation du prop-2-yl-sulfate de clopidogrel, de formule suivante : 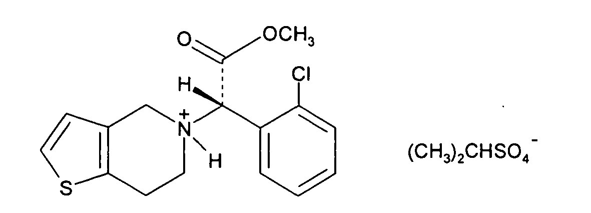 ou du perchlorate de clopidogrel, de formule suivante : 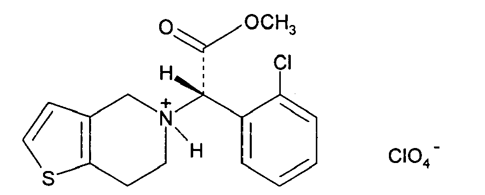 en tant que produit intermédiaire dans la préparation de la forme 1 de l'hydrogénosulfate de clopidogrel.

12. Utilisation conforme à la revendication 11, dans laquelle on effectue la cristallisation suivant un procédé conforme à l'une des revendications 1 à 7.

13. Procédé de préparation de la forme 1 de l'hydrogénosulfate de clopidogrel, dans lequel on utilise, en tant que produit intermédiaire, du prop-2-yl-sulfate de clopidogrel, de formule suivante : 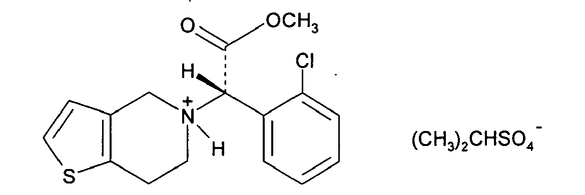 ou du perchlorate de clopidogrel, de formule suivante :

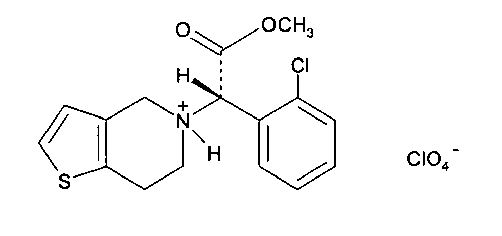

14. Procédé conforme à la revendication 13, dans lequel on effectue la cristallisation suivant un procédé conforme à l'une des revendications 1 à 7.
